# EUROPEAN PATENT SPECIFICATION

(11) **EP 4 348 444 B1**
(45) Date of publication and mention of the grant of the patent: **29.10.2025**
(21) Application number: 22731391.3
(22) Date of filing: 20.05.2022
(51) Int. Cl.: G06F 16/25, G06F 16/81, G06F 16/34

(54) **TECHNIQUES FOR ABSTRACTION OF UNSTRUCTURED CLINICAL TRIAL HEALTH DATA**
VERFAHREN ZUR ABSTRAKTION UNSTRUKTURIERTER GESUNDHEITSDATEN FÜR KLINISCHE STUDIEN
TECHNIQUES D'ABSTRACTION DE DONNÉES DE SANTÉ D'ESSAI CLINIQUE NON STRUCTURÉES

(30) Priority: 27.05.2021 US 202163193659 P
(43) Date of publication of application: 10.04.2024
(73) Proprietor: GENENTECH, INC., South San Francisco, CA 94080 (US)
(72) Inventor: COREY, Lukas Robert, San Francisco, CA 94080 (US); CRAWFORD, Jennifer Renee, San Francisco, CA 94080 (US); DOUGLAS, Connor Patrick, San Francisco, CA 94080 (US); FERNANDEZ, Benjamin Pamandanan, San Francisco, CA 94080 (US)
(74) Representative: Mewburn Ellis LLP
(86) International application number: PCT/US2022/030369
(87) International publication number: WO 2022/251076

(56) References cited:
- US-A1- 2005 267 871
- US-A1- 2006 005 139
- US-A1- 2020 176 098
- US-B1- 10 789 461

## Description

### CROSS-REFERENCE TO RELATED APPLICATIONS

This application claims the benefit of and priority to U.S. Provisional Patent Application Numbers: 63/193,659 (filed on May 27, 2021).

### FIELD

The present disclosure relates to clinical trial health data abstraction, and in particular to abstraction of unstructured clinical trial health data into structured clinical trial health data.

### BACKGROUND

**In** healthcare, clinical trials help answer questions related to medical treatments and interventions. For example, clinical trials for new drug treatments determine efficacy, safety, dosage, side effects, and other information related to using a drug treatment. Subjects enroll in clinical trials and have health data collected throughout the clinical trial that is analyzed to determine the outcomes. A protocol document is created to outline how the clinical trial is to be conducted. For example, the protocol document can include objectives, design, methodology, and safety aspects for the clinical trial. Portions of a protocol document may be usable in multiple clinical trials. However, protocol documents are typically unstructured documents that cannot be easily analyzed or reused. US2020176098A1 discloses extracting concepts from a text of a medical document.

### SUMMARY

The invention is defined by the features of the independent claims. Further embodiments are the subject-matter of the dependent claims. In various embodiments, a computer-implemented method is provided that involves obtaining, by a data transformation system, an unstructured document that includes various types of content for a given event (e.g., clinical trial). The method further involves converting, by the data transformation system, the unstructured document from an original format (e.g., Extensible Markup Language (XML)) into a standardized format (e.g., Hypertext Markup Language (HTML)). The method further involves processing, by the data transformation system, the unstructured document in the standardized format to identify template-mapped content data and processing the template-mapped content data to identify high-interest content data. The method further involves generating, by the data transformation system, a dictionary data structure for each of the template-mapped content data and the high-interest content data. The dictionary data structure includes key-value pairs. The method further involves generating, by the data transformation system, a structured document based on the dictionary data structure for each of the template-mapped content data and the high-interest content data. Generating the structured document includes serializing the key-value pairs within the dictionary data structure to data objects in an open standard file format (e.g., JavaScript Open Notation (JSON)).

In some embodiments, obtaining the unstructured document involves obtaining a plurality of unstructured documents for the given event, collating protocol documents and amendment history documents from the plurality of unstructured documents, and retrieving the unstructured document from the collated protocol documents.

In some embodiments, collating the protocol documents and the amendment history documents involves identifying the protocol documents and the amendment history documents within the plurality of unstructured documents by matching a key word or phrase associated with the protocol documents or the amendment history documents to a word or phrase within a name or content of an unstructured document. Collating the protocol documents and the amendment history documents further involves grouping the identified protocol documents and the amendment history documents into a subgroup of unstructured documents.

In some embodiments, converting the unstructured document into the standardized format involves extracting a list of tags from the unstructured document based on a source markup language (e.g., XML) used to program the unstructured document. The list of tags shows a structure of content in the unstructured document. The content includes headers, text, tables, images, or any combination thereof. Converting the unstructured document into the standardized format further involves mapping the list of tags to a corresponding list of tags that are defined by a target markup language (e.g., HTML) and converting the content of the unstructured document from the original format into the standardized format based on the mapping between the list of tags and the corresponding list of tags defined by the target markup language.

In some embodiments, the method further involves extracting, by the data transformation system, the corresponding list of tags defined by the target markup language from the unstructured document in the standardized format and removing redundant tags and associated content.

In some embodiments, the processing to identify the template-mapped content data involves determining whether each element in the unstructured document is a header, text, a table, or an image. In response to determining the element is a header, the method involves determining a nesting level of the header and storing the header as a subheader under a header or as an independent header based on the nesting level. In response to determining the element is the text, the table, or the image, the method involves storing the text, the table, or the image under a latest processed header.

In some embodiments, generating the dictionary data structure for the template-mapped content data involves writing the template-mapped content data in the dictionary data structure as the key-value pairs. The headers are written as keys, and the subheaders, the text, the tables, and the images under the headers are written as values associated with the keys. The serializing the key-value pairs involves writing the key-value pairs to an object-based data structure comprising the data objects with properties, the properties are the key-value pairs, each of the keys is a name of a property, and each of the values is a schema used to validate the property.

In some embodiments, the processing to identify the high-interest content data involves parsing each table within the template-mapped content data into a list of table elements and searching the list of table elements to find an element associated with high-interest content. In response to finding the element, the method involves searching the list of table elements to find another element associated with orientation of the table. In response to finding another element, the method involves determining the orientation of the table and analyzing the table elements based on the orientation to identify procedures and cell content. The method further involves storing the identified procedures and the cell content.

In some embodiments, generating the dictionary data structure for the high-interest content data involves writing the high-interest content data in the dictionary data structure as the key-value pairs. The procedures are written as keys, and the cell content are written as values associated with the keys. The serializing the key-value pairs involves writing the key-value pairs to an object based data structure comprising the data objects with properties, the properties are the key-value pairs, each of the keys is a name of a property, and each of the values is a schema used to validate the property.

In some embodiments, analyzing the table elements is performed using a statistical based approach or a natural language processing model.

In some embodiments, the method further involves processing, by the data transformation system, the unstructured document to identify non-template-mapped content data and generating the dictionary data structure for the non-template-mapped content data. The structured document is generated based on the non-template-mapped content data, the template-mapped content data, and the high-interest content data.

In some embodiments, processing the unstructured document to identify the non-template-mapped content data involves parsing the unstructured document to find a header-style paragraph containing a key word or phrase associated with non-template-mapped content. In response to finding the header-style paragraph, the method involves collecting the non-template-mapped content under the header-style paragraph until a style change in the non-template-mapped content is identified or another header-style paragraph is found. The method further involves storing an identifier for the given event, the header-style paragraphs, and the non-template-mapped content in a list as the non-template-mapped content data.

In some embodiments, generating the dictionary data structure for the non-template-mapped content data involves writing the non-template-mapped content data in the dictionary data structure as the key-value pairs, wherein the identifier for the given event and the header-style paragraphs are written as keys, and the non-template-mapped content under the header-style paragraphs are written as values associated with the keys. The serializing the key-value pairs involves writing the key-value pairs to an object based data structure including the data objects with properties. The properties are the key-value pairs, each of the keys is a name of a property, and each of the values is a schema used to validate the property.

In some embodiments, the method further involves selecting, by the data transformation system, another unstructured document from the amendment history documents and processing the another unstructured document to identify non-template-mapped content data. The method further involves generating, by the data transformation system, the dictionary data structure for the non-template-mapped content data and generating another structured document based on the non-template-mapped content data. Generating the another structured document involves serializing the key-value pairs within the dictionary data structure to the data objects in the open standard file format.

In some embodiments, the method further involves communicating, by the data transformation system, the structured document to an end user.

In some embodiments, the method further involves providing, by the data transformation system, the structured document to a search engine and performing, by the search engine, a query for data using the structured document.

In some embodiments, the method further involves providing, by the data transformation system, the structured document to an authoring tool and generating, by the authoring tool, a new document based on the template-mapped content data, the high-interest content data, the non-template-mapped content data, or any combination thereof.

In some embodiments, the method further involves providing, by the data transformation system, the structured document to an analysis tool and analyzing, by the analysis tool, the template-mapped content data, the high-interest content data, the non-template-mapped content data, or any combination thereof to determine an insight.

In some embodiments, a system is provided that includes one or more data processors and a non-transitory computer readable storage medium containing instructions which, when executed on the one or more data processors, cause the one or more data processors to perform part or all of one or more methods disclosed herein.

In some embodiments, a computer-program product is provided that is tangibly embodied in a non-transitory machine-readable storage medium and that includes instructions configured to cause one or more data processors to perform part or all of one or more methods disclosed herein.

Some embodiments of the present disclosure include a system including one or more data processors. In some embodiments, the system includes a non-transitory computer readable storage medium containing instructions which, when executed on the one or more data processors, cause the one or more data processors to perform part or all of one or more methods and/or part or all of one or more processes disclosed herein. Some embodiments of the present disclosure include a computer-program product tangibly embodied in a non-transitory machine-readable storage medium, including instructions configured to cause one or more data processors to perform part or all of one or more methods and/or part or all of one or more processes disclosed herein.

### BRIEF DESCRIPTION OF THE DRAWINGS

The present disclosure is described in conjunction with the appended figures:
FIG. 1 depicts a block diagram of a computing environment for providing abstraction of clinical trial health data according to various embodiments;
FIG. 2 depicts a block diagram of an example of a template-mapped content data object according to various embodiments;
FIG. 3 depicts a block diagram of a process for standardizing a format of an unstructured document according to various embodiments;
FIG. 4 depicts a block diagram of a process for identifying template-mapped content data and high-interest content data according to various embodiments;
FIG. 5 depicts a block diagram of a process for identifying non-template-mapped content data according to various embodiments; and
FIG. 6 depicts a flowchart illustrating a process for abstraction of unstructured clinical trial health data according to various embodiments.

In the appended figures, similar components and/or features can have the same reference label. Further, various components of the same type can be distinguished by following the reference label by a dash and a second label that distinguishes among the similar components. If only the first reference label is used in the specification, the description is applicable to any one of the similar components having the same first reference label irrespective of the second reference label.

### DETAILED DESCRIPTION

### I. Overview

The present disclosure describes techniques for abstracting unstructured clinical trial health data and converting it into a structured document. More specifically, embodiments of the present disclosure provide techniques for obtaining, by a data transformation system, health data for a given event (e.g., a clinical trial) in an unstructured document and generating a structured document based on various types of content in the unstructured document.

Protocol documents and amendment history documents for various purposes, such as a clinical trial, are typically generated as unstructured documents. Unstructured documents may be difficult to analyze and reuse in future clinical trials. Since clinical trials may only include minor changes between each other, it can be tedious and time consuming for a clinical trial coordinator to generate a new protocol document for each clinical trial. In any given section of a protocol document, the information may be substantially different protocol to protocol, but the overall structure of the protocol document may not shift much. However, the content of one section being similar to one protocol ('A') does not necessarily mean that the content of another section is similar to the content of that section in protocol ('A') and for that other section another protocol ('B') may have similar content. As such, traditional organization search strategies of grouping past-used protocols together by disease area, treatment type, hospital, subject age, or other characteristics may not provide a meaningful benefit in identifying related protocol documents and/or constructing new protocol documents. Therefore, abstraction helps map content to generate better storage and search capabilities for analysis and construction of protocol documents. It may also be time consuming for the clinical trial coordinator to manually search the protocol documents for a particular piece of data.

To address these limitations and problems, the techniques for clinical trial health data abstraction in the present disclosure utilize an accurate and automated approach to generate a structured document from an unstructured document. This technique is intended to obtain, by a data transformation system, an unstructured document for a given event, identify template-mapped content data, non-template-mapped content data, and high-interest content data. The template-mapped content is content in which there exists an internal organization standard to map it one to one (1:1) against (e.g., a background section). A clinical trial coordinator of the organization defines and generates the standard for which the template-mapped content is mapped against. High-interest content is content in which there exists an internal organization standard to map it against, but consumers of the data have requested additional features to augment the content with other than the 1:1 mapping for template-mapped content (e.g., grouping data for inclusion/exclusion criteria). The non-template-mapped content is content in which there does not exist an internal organization standard to map it 1:1 against (e.g., an amendment section). This technique generates a data structure for each of the various types of content, and a structured document from the dictionary data structures. Some or all of the structured document may then be easily searched, analyzed, or reused.

One illustrative embodiment of the present disclosure is directed to a method that includes obtaining, by a data transformation system, an unstructured document that includes various types of content for a given event. The method further includes converting, by the data transformation system, the unstructured document from an original format (e.g., Extensible Markup Language (XML)) into a standardized format (e.g., Hypertext Markup Language (HTML)). The method further includes processing, by the data transformation system, the unstructured document in the standardized format to identify template-mapped content data and processing the template-mapped content data to identify high-interest content data. The method further includes generating, by the data transformation system, a dictionary data structure for each of the template-mapped content data and the high-interest content data. The dictionary data structure includes key-value pairs. The method further includes generating, by the data transformation system, a structured document based on the dictionary data structure for each of the template-mapped content data and the high-interest content data. Generating the structured document includes serializing the key-value pairs within the dictionary data structure to data objects in an open standard file format (e.g., JavaScript Open Notation (JSON)).

In some instances, the method further includes processing, by the data transformation system, the unstructured document to identify non-template-mapped content data and generating the dictionary data structure for the non-template-mapped content data. The structured document is generated based on the non-template-mapped content data, the template-mapped content data, and the high-interest content data.

In some instances, the method further includes communicating, by the data transformation system, the structured document to an end user. The method may also include providing the structured document to a search engine and performing, by the search engine, a query for data using the structured document. Additionally or alternatively, the method may include providing, by the data transformation system, the structured document to an authoring tool and generating, by the authoring tool, a new document based on the template-mapped content data, the high-interest content data, the non-template-mapped content data, or any combination thereof. In some instances, the method further includes providing, by the data transformation system, the structured document to an analysis tool and analyzing, by the analysis tool, the template-mapped content data, the high-interest content data, the non-template-mapped content data, or any combination thereof to determine an insight.

### II. Example Computing Environment

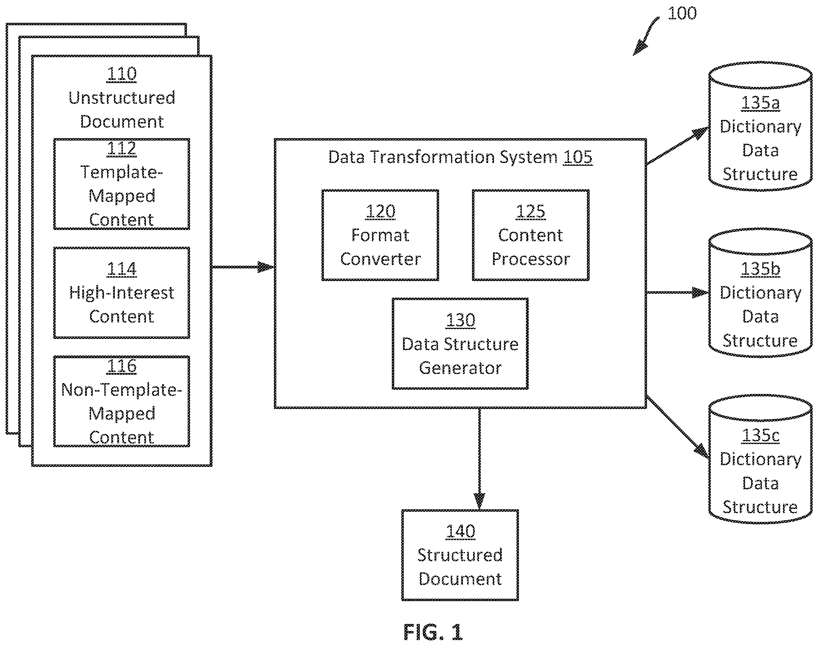

FIG. 1 depicts a block diagram of a computing environment 100 for providing abstraction of clinical trial health data according to various embodiments. In the illustrated embodiment, computing environment 100 includes a data transformation system 105 in communication with dictionary data structures 135a-c. The data transformation system 105 also receives an unstructured document 110 and generates a structured document 140, which may be on a local or remote computing system to the data transformation system 105. The components of the computing environment 100 may communicate via a communication network. The communication network can be any type of network familiar to those skilled in the art that can support data communications using any of a variety of available protocols, including without limitation TCP/IP (transmission control protocol/Internet protocol), SNA (systems network architecture), IPX (Internet packet exchange), AppleTalk^{®}, and the like. Merely by way of example, network(s) can be a local area network (LAN), networks based on Ethernet, Token-Ring, a wide-area network (WAN), the Internet, a virtual network, a virtual private network (VPN), an intranet, an extranet, a public switched telephone network (PSTN), an infra-red network, a wireless network (e.g., a network operating under any of the Institute of Electrical and Electronics (IEEE) 1002.11 suite of protocols, Bluetooth^{®}, and/or any other wireless protocol), and/or any combination of these and/or other networks.

The unstructured document 110 can be a protocol document that includes various types of content for a given event. The event corresponds to a clinical trial or other health-related event. If the unstructured document 110 is a protocol document for a clinical trial, the unstructured document 110 can describe how the clinical trial is to be conducted. For example, the protocol can include objectives, a design, methodology, and organization of the clinical trial. The types of content can include template-mapped content 112, high-interest content 114, and non-template-mapped content 116. Background section content can be the template-mapped content 112, a Schedule of Assessments (SOA), which is a table that can be further processed by parsing out the individual rows or study visits through rules-based logic, can be the high-interest content 114, and an amendment section, which takes on many different forms from protocol to protocol, can be included in the non-template-mapped content 116.

In some instances, the data transformation system 105 includes a format converter 120. The format converter 120 can obtain the unstructured document 110 that includes the various types of content for the given event (e.g., clinical trial). The unstructured document 110 can also be in a particular format that is not standard. For example, the unstructured document 110 may be in an Extensible Markup Language (XML) and a Hypertext Markup Language can be considered a standardized format. The format converter 120 can convert the unstructured document 110 in the original format to the standardized format. The standardized format allows predefined tags to be used and parsed that may not exist in the original format. As such, a structured document can be easily generated from the standardized format.

A content processor 125 of the data transformation system 105 processes the unstructured document 110 in the standardized format to identify template-mapped content data, which corresponds to the template-mapped content 112. The template-mapped content data may correspond to headers, text, tables, or images of the unstructured document 110. For example, the template-mapped content data can be a background section of the unstructured document 110 and the content processor 125 can process the unstructured document 110 to identify the background section. If the unstructured document 110 remains in the original format, the data transformation system 105 can similarly processes the unstructured document 110 to identify template-mapped content data. Indicators of the template-mapped content data may be different in the original format than in the standardized format, but a mapping can be generated from either format to identify the template-mapped content data.

In some instances, the content processor 125 also processes the template-mapped content data to identify high-interest content data in the unstructured document 110. The content processor 125 can search table elements of the tables of the template-mapped content data, or other elements of the template-mapped content data, to find elements associated with the high-interest content 114. For example, objectives and endpoints of the clinical trial can be included in the high-interest content 114, and the content processor 125 can identify elements associated with the objectives and endpoints.

The content processor 125 also processes the unstructured document 110 to identify non-template-mapped content data. The non-template-mapped content 116 can include notes on aspects of the protocol and amendments to the protocol. The content processor 125 can identify elements of the unstructured document 110 that corresponds to the notes and the amendments to identify the non-template-mapped content data.

A data structure generator 130 of the data transformation system 105 generates the dictionary data structures 135a-c for the template-mapped content data, the high-interest content data, and the non-template-mapped content data. Each of the dictionary data structures 135a-c can correspond to one of the types of content. For example, the dictionary data structure 135a can be for the template-mapped content data, the dictionary data structure 135b can be for the high-interest content data, and the dictionary data structure 135c can be for the non-template-mapped content data. In other examples, the data structure generator 130 can generate one data structure that includes all of the types of content. The dictionary data structures 135a-c can include key-value pairs for each of the types of content.

The data transformation system 105 generates a structured document 140 based on the dictionary data structures 135a-c. The data transformation system 105 can serialize the key-value pairs within the dictionary data structures 135a-c to data objects in an open standard file format. For example, the key-value pairs can be serialized to JavaScript Open Notation (JSON) data objects. The structured document 140 can be organized and formatted so that it is easily searchable and desired content can be easily located.

The data transformation system 105 can communicate the structured document 140 to an end user. The end user may be a coordinator of the clinical trial or a coordinator of another clinical trial. The end user can use the structured document 140 to generate a new protocol that reuses content from the structured document 140. The data transformation system 105 may additionally or alternatively provide the structured document 140 to a search engine. The search engine can then perform a query for data using the structured document 140 based on an input query. For example, the search engine can perform a query on the structured document 140 for data associated with an SOA table for the clinical trial. In some instances, the data transformation system 105 can provide the structured document 140 to an analysis tool that can analyze one or more of the template-mapped content data, the high-interest content data, and the non-template-mapped content data to determine an insight. For example, the insight may relate to a patient burden and Fair Market Value analysis through high-interest content data of an SOA. The data transformation system 105 may additionally or alternatively provide the structured document 140 to an authoring tool that generates a new document based on the template-mapped content data, the high-interest content data, the non-template-mapped content data, or any combination thereof. The new document may include new content as well as the template-mapped content data, the high-interest content data, the non-template-mapped content data, or the combination thereof. III. Dictionary Data Structure

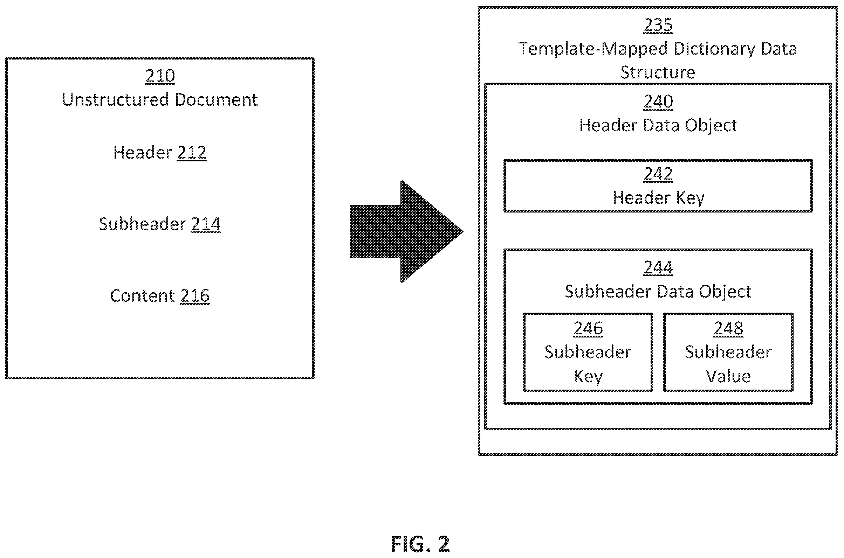

FIG. 2 depicts a block diagram of an example of a template-mapped dictionary data structure 235 according to various embodiments. The template-mapped dictionary data structure 235 can be generated from an unstructured document 210 by a data transformation system (e.g., the data transformation system 105 in FIG. 1).

The unstructured document 210 can include template-mapped content of a header 212, a subheader 214, and content 216 under the subheader 214. For example, the header 212 can be "Background", the subheader 214 can be "Previous Clinical Trial Findings", and the content 216 can be text describing findings of previous clinical trials. A header is a header that has no parent headers above it in the document hierarchy, whereas a subheader is a header with a parent header above it.

In some instances, the data transformation system 105 generates key-value pairs for the template-mapped content. The template-mapped content can then be written to the template-mapped dictionary data structure 235 as the key-value pairs. The template-mapped dictionary data structure 235 can include a header data object 240 and a subheader data object 244. The header key 242 for the header data object 240 can be the header 212, and the value for the header data object 240 can be the subheader data object 244. The subheader key 246 for the subheader data object 244 can be the subheader 214, and a subheader value 248 for the subheader data object 244 can be the content 216. This structure is recursive, such that if there is a subheader of the subheader data object 244, that information is stored in another key-value pair of the subheader data object 244.

### IV. Methods

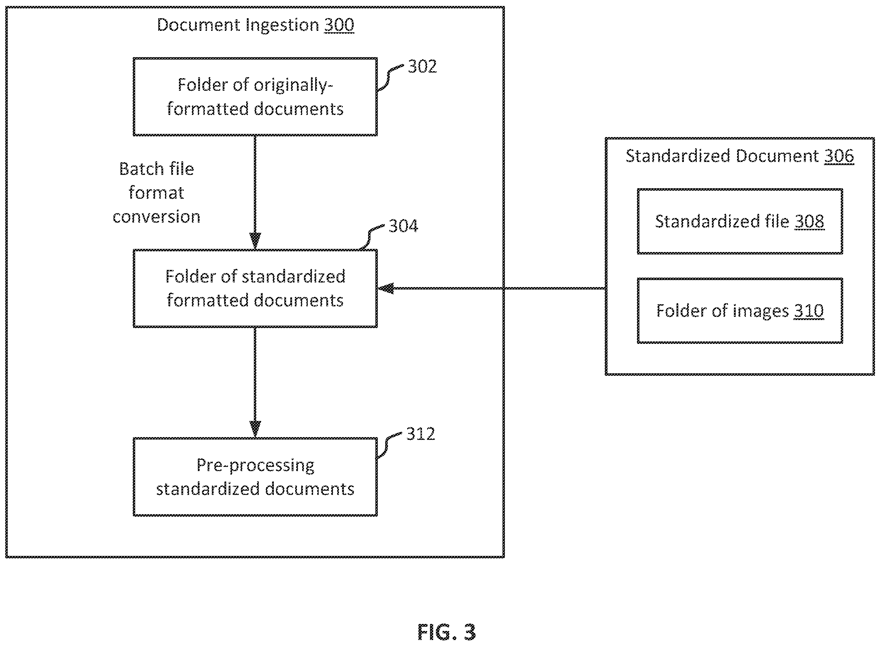

FIGS. 3-6 illustrate processes for abstraction of unstructured clinical trial health data. The processes depicted in FIGS. 3-6 are implemented by the architecture, systems, and techniques depicted in FIGS. 1 and 2. Individual embodiments may be described as a process which is depicted as a flowchart, a flow diagram, a data flow diagram, a structure diagram, or a block diagram. Although a flowchart may describe the operations as a sequential process, many of the operations may be performed in parallel or concurrently. In addition, the order of the operations may be rearranged. A process is terminated when its operations are completed, but could have additional steps not included in a figure. A process may correspond to a method, a function, a procedure, a subroutine, a subprogram, etc. When a process corresponds to a function, its termination may correspond to a return of the function to the calling function or the main function.

The processes and/or operations depicted in FIGS. 3-6 may be implemented in software (e.g., code, instructions, program) executed by one or more processing units (e.g., processors cores), hardware, or combinations thereof. The software may be stored in a memory (e.g., on a memory device, on a non-transitory computer-readable storage medium). The particular series of processing steps in FIGS. 3-6 are not intended to be limiting. Other sequences of steps may also be performed according to alternative embodiments. For example, in alternative embodiments the steps outlined above may be performed in a different order. Moreover, the individual steps illustrated in FIGS. 3-6 may include multiple sub-steps that may be performed in various sequences as appropriate to the individual step. Furthermore, additional steps may be added or removed depending on the particular applications. One of ordinary skill in the art would recognize many variations, modifications, and alternatives.

FIG. 3 depicts a block diagram of a process for standardizing a format of an unstructured document according to various embodiments. Document ingestion 300 can be performed by the data transformation system 105. The document ingestion 300 can include a first folder 302 of unstructured documents in an original format. Each of the unstructured documents in the first folder 302 can be associated with the given event. For example, the unstructured documents can include protocol documents, amendment history documents, and other documents for a clinical trial. Protocol documents and amendment history documents can be collated from the unstructured documents. The data transformation system 105 can identify the protocol documents and the amendment history documents within the first folder 302 by matching a key word or phrase associated with the protocol documents or the amendment history documents to a word or phrase within a name or content of an unstructured document. For example, the word "amendment" being in a title or header can indicate the unstructured document 110 is an amendment history document. An unstructured document 110 can then be retrieved from the collated protocol documents to be processed into a structured document 140.

In some instances, the document ingestion 300 involves batch file format conversion from an original format to a standardized format. The unstructured documents in the first folder 302 can be in the original format that is different from the standardized format. For example, the original format, or source markup language, can be XML and the standardized format, or target markup language, can be HTML. The data transformation system 105 can perform the conversion by extracting a list of tags from the unstructured document 110 based on the source markup language that is used to program the unstructured document 110. The list of tags shows a structure of content in the unstructured document 110. The data transformation system 105 maps the list of tags to a corresponding list of tags that are defined by the target markup language. For example, a "<content>" tag in XML can be mapped to a "<body>" tag in HTML. The data transformation system 105 then converts the content of the unstructured document 110 from the original format into the standardized format based on the mapping between the list of tags and the corresponding list of tags defined by the target markup language. For instance, the text under the "<content>" tag in XML can be converted into the HTML under the "<body>" tag.

The unstructured documents in the standardized format can be stored in a second folder 304. A standardized document 306 in the second folder 304 can include a standardized file 308 and a folder of images 310. The standardized file 308 can include the text content of the unstructured document 110 in the standardized format and the folder of images 310 can include images from the unstructured document 110.

In some instances, the document ingestion 300 also includes pre-processing 312 the unstructured documents in the standardized format. The pre-processing 312 can involve standardizing the target markup language tags. For example, the data transformation system 105 can perform font standardization and/or compression on the standardized documents. The pre-processing 312 may also include grouping identified protocol documents and amendment history documents into a subgroup of unstructured documents.

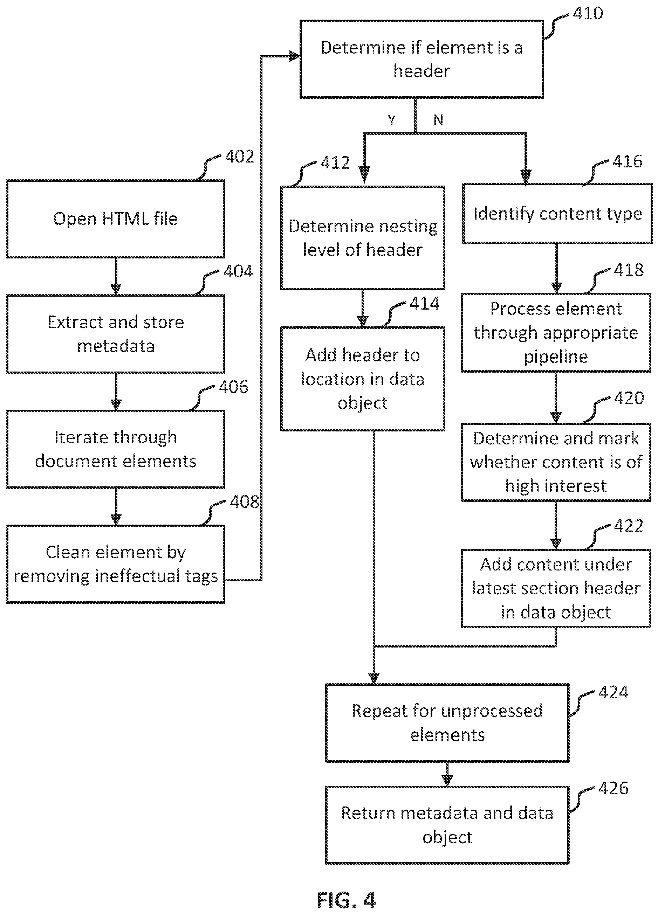

FIG. 4 depicts a block diagram of a process for identifying template-mapped content data and high-interest content data according to various embodiments. At block 402, the data transformation system can open an HTML file. The HTML file can correspond to an unstructured document 110, such as a protocol document, in a standardized form.

At block 404, the data transformation system 105 can extract and store metadata of the unstructured document 110. The metadata includes a title of the unstructured document 110, a date of creation and last-modified date for the unstructured document 110, an author of the unstructured document 110, etc. The data transformation system 105 can identify the metadata based on HTML tags associated with metadata. The metadata can be stored so that it can be incorporated into a structured document 140 that is generated from the unstructured document 110.

At block 406, the data transformation system 105 can iterate through each element of the unstructured document 110. Each element can include one or more HTML tags and can correspond to either a header, text, a table, or an image.

At block 408, the data transformation system 105 can clean an element by removing ineffectual tags. The ineffectual tags are removed from the unstructured document 110 in a standardized format. An ineffectual tag can be a redundant tag or a tag that does not provide information needed in converting the unstructured document 110 into a structured document 140. Content associated with the ineffectual tags may also be removed from the element.

At block 410, the data transformation system 105 can determine whether an element is a header. The data transformation system 105 can determine whether a tag associated with the element corresponds to a tag for a header that is predefined as a header in a list of possible tags in the standardized format. If the tag corresponds to a header, the process proceeds to block 412. Otherwise, the process proceeds to block 416.

At block 412, the data transformation system 105 can determine a nesting level of the header. A header without a parent header above it has a higher nesting level than a header with a parent header above it. The data transformation system 105 can use the tags to determine the nesting level of the header.

At block 414, the data transformation system 105 adds the header to a location in the data object. If the header is a subheader, it can be stored under another header in the data object. If the header is not a subheader, it can be stored as an independent header in the data object. The data object can be in a dictionary data structure 135 of key-value pairs. An independent header can be stored as the key, a subheader for the header can be stored as another data object within the data object and can be another key, and content under the subheader can be stored as the value for the other data object. If the header does not include a subheader, the header can be the key and the content under the header can be the value.

At block 416, the data transformation system 105 can identify a content type for the element. The content type can correspond to text, a table, or an image. The data transformation system 105 may use the tags of the unstructured document 110 in the standardized format to identify the content type.

At block 418, the data transformation system 105 can process the element through an appropriate pipeline. For text, the pipeline can involve determining whether the text occurs in a Table of Contents of the unstructured document 110. The data transformation system 105 can use a rules-based approach to determine whether the text occurs in the Table of Contents. For example, the text being within a heading titled "Table of Contents" indicates the text is in a Table of Contents. Additionally, the text occurring within a predefined portion (e.g., the first 10 pages) of the unstructured document 110 can indicate the text occurs in a Table of Contents. If the text occurs in the Table of Contents, the pipeline involves determining whether the text indicates a section, Appendix, table, or image that occurs in the unstructured document. If the text indicates a section, Appendix, table, or image, the pipeline can involve parsing the name and title of the item. For example, the text can include a name of "Table 3" and a title of "Schedule of Assessments". The item name and title can be stored in a Table of Contents dictionary. If the text does not list a section, Appendix, table, or image, the pipeline can involve determining whether the text is a header based on indications associated with the text. For example, a tag may indicate the text is a header, or font characteristics, such as bold and underlines, can indicate the text is a header. If the text is a header, the relevant title and section information can be stored. Otherwise, the data transformation system 105 can determine whether the text occurs sufficiently soon after a table or image. If the text does occur sufficiently soon after a table or image, a standard protocol footnote/note/key format can be used to determine if the text contains footnotes, notes, or keys. If the text contains footnotes, notes, or keys, they can be stored under a latest processed table or image information.

In some instances, the pipeline for tables involves determining if the table includes a nested table. If the data transformation system 105 finds another table before reaching an end of a first table, the data transformation system 105 determines that the table includes a nested table. If the table includes a nested table, the data transformation system 105 can determine whether the nested structure is programmatically fixable. The table is fixed to remove the nested table from the table. The nested table may be subsequently parsed as a separate table. If the table does not include a nested table, or after the table is fixed, the pipeline can involve parsing the table content into a list of lists and storing a data object. The pipeline can also involve documenting possible errors that occurred during table processing and setting the table title to a latest processed title. If there is no latest processed title, the page header can be checked for the title. The table header name can be set to the latest processed header name found. If no latest header name is found, the page header can be checked for the header name. The gathered table information can then be stored.

In some instances, the pipeline for images involves checking if the image contains a source file. If the image does not include a source file, an indication can be added that the image lacks a source file. If the image does include a source file, or once the indication is added, the image title can be set to the latest processed title. If no latest processed title exists, the page header can be checked for the title. The image header name can be set to the latest processed header name found. If no latest header name is found, the page header can be checked for the header name. The gathered image information can then be stored.

The stored text, table information, and image information can be template-mapped content data that may include high-interest content data. The template-mapped content can be further processed to identify the high-interest content data. At block 420, the data transformation system 105 can determine and mark whether content is of high interest. For example, the data transformation system 105 can parse each table within the template-mapped content data into a list of table elements. The list of table elements can then be searched to find an element associated with high-interest content, such as an element associated with an SOA table (e.g., cells including a positive study visit indication, such as by "X"s or another character). Once the element is found, the list of table elements can be searched again to find another element associated with orientation of the table. String matching between cell text and known SOA header words (e.g., "cycle" or "week") can be used to determine the orientation of the table. Procedures can then be identified and extracted by analyzing the table elements based on the orientation of the table. Analyzing the table may be performed using a statistical based approach or a natural language processing model. For example, named-entity recognition may be used in extracting named procedures within the text of the unstructured document 110. Cell content can also be identified and extracted, such as a study visit count for each procedure. The identified procedures and cell content can then be stored.

At block 422, the data transformation system 105 can add the content under the latest processed section header in a data object 240. The data object 240 can be included in a dictionary data structure 235. For the template-mapped content data, the dictionary data structure 235 can be generated by writing the template-mapped content data in the dictionary data structure 235 as key-value pairs. Headers can be written as keys and subheaders, text, tables, and images under the headers can be written as values associated with the keys. The subheaders may be a value for a header data object and a key for a subheader data object, as described in FIG. 2. The key-value pairs can be serialized by writing the key-value pairs to an object based data structure that includes data objects (e.g., JSON objects) with properties. The properties can be the key-value pairs, with each key being a name of a property and each of the values being a schema used to validate the property. For example, the schema can indicate the value is to include particular parameters and be a string. The property can be validated if a value includes the particular parameters and is expressed as a string.

Similar to template-mapped content data, a dictionary data structure for the high-interest content data can be generated by writing the high-interest content data in the dictionary data structure as the key-value pairs. The dictionary data structure for high-interest content can be similar to the template-mapped content dictionary data structure 235, but procedures can be written as keys and cell content can be written as values associated with the keys. The key-value pairs can be serialized by writing the key-value pairs to an object based data structure that includes data objects with properties. The properties can be the key-value pairs, with each key being a name of a property and each of the values being a schema used to validate the property. The data objects for the template-mapped content data and the high-interest content data can be stored as a list in the dictionary data structure.

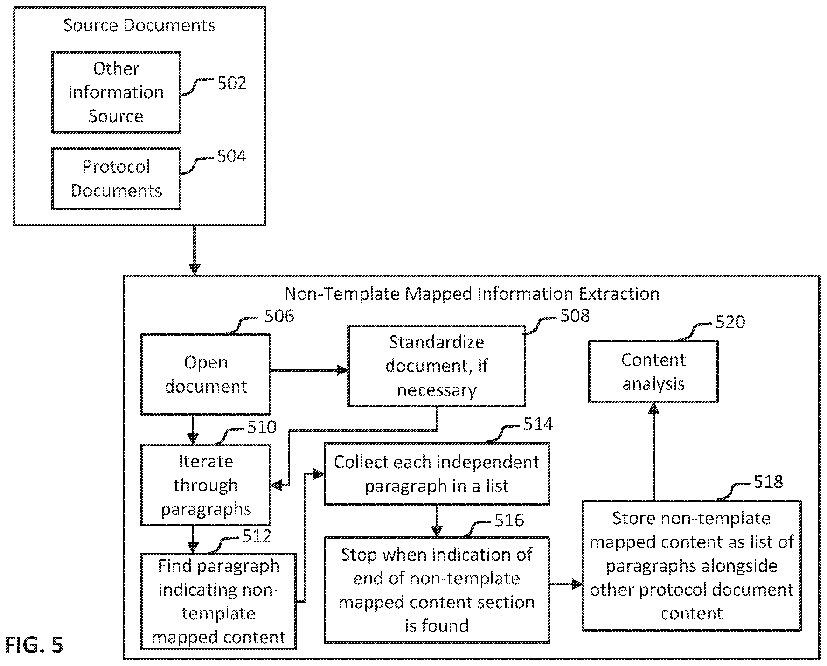

At block 424, the data transformation system 105 can repeat the processing for unprocessed elements. As a result, each element of the unstructured document 110 can be identified as template-mapped content data, high-interest content data, or non-template-mapped content data (further described in FIG. 5). The processed elements can be stored as data objects in a dictionary data structure that can be aggregated into a structured document 140.

At block 426, the data transformation system 105 can return the metadata and a data object. The data transformation system 105 may return more than one data object, and optionally, an aggregation of the data objects as a structured document 140 that can be used for searching for data, data analysis, or protocol generation by a clinical trial coordinator.

FIG. 5 depicts a block diagram of a process for identifying non-template-mapped content data according to various embodiments. Examples of source documents that can include non-template-mapped content data include protocol documents 504 and other information sources 502, such as amendment history documents. Amendment history documents may only provide non-template-mapped content data, whereas the protocol documents 504 can provide non-template-mapped content data, template-mapped content data, and high-interest content data.

At block 506, the data transformation system 105 can open a document. The document can be an unstructured protocol document or an unstructured amendment history document and may be in a standardized format, such as HTML. If the document is in an original, non-standardized format, the process can proceed to block 508. Otherwise, the process can proceed to block 510.

At block 508, the data transformation system 105 can standardize the unstructured document 110. The conversion can include the process as described in FIG. 3.

At block 510, the data transformation system 105 can iterate through paragraphs of the unstructured document 110 in the standardized format until, at block 512, the data transformation system 105 finds a paragraph indicating non-template mapped content 116. The paragraph can be a header-style paragraph that contains a key word or phrase associated with non-template-mapped content 116. The key word or phrase may be included in a tag of the list of tags for the standardized format. Content associated with particular tags in the list of tags that correspond to non-template mapped content is determined to be non-template-mapped content 116. For example, the key word or phrase may be "amendment" or "summary of changes", or a similar word or phrase.

At block 514, the data transformation system 105 can collect each independent paragraph in a list. The list can be collected under the header-style paragraph until, at block 516, a style change in the non-template-mapped content 116 is identified, or another header-style paragraph is found. For example, a space break may be a style change that indicates an end of the non-template-mapped content 116.

At block 518, the non-template-mapped content 116 can be stored as a list of paragraphs alongside other protocol document content (if the source document is one of the protocol document 504). An identifier for the given event and the header-style paragraphs can be stored with the non-template-mapped content 116 in the list. The identifier, the header-style paragraphs, and the non-template-mapped content 116 together can be the non-template-mapped content data.

In some instances, a dictionary data structure 135c can be generated for the non-template-mapped content 116. The dictionary data structure 135c can be generated by writing the non-template-mapped content data in the dictionary data structure 135c as key-value pairs. The dictionary data structure 135c for the non-template-mapped content 116 can be similar to the template-mapped content dictionary data structure 235, but the identifier for the given event and the header-style paragraphs can be written as keys and the non-template-mapped content under the header-style paragraphs can be written as values associated with the keys. The key-value pairs can be serialized to data objects in an open standard file format by writing the key-value pairs to an object based data structure that includes data objects with properties. The properties can be the key-value pairs, with each key being a name of a property and each of the values being a schema used to validate the property. The data objects for the non-template mapped content data can be stored as a list in the dictionary data structure 135c.

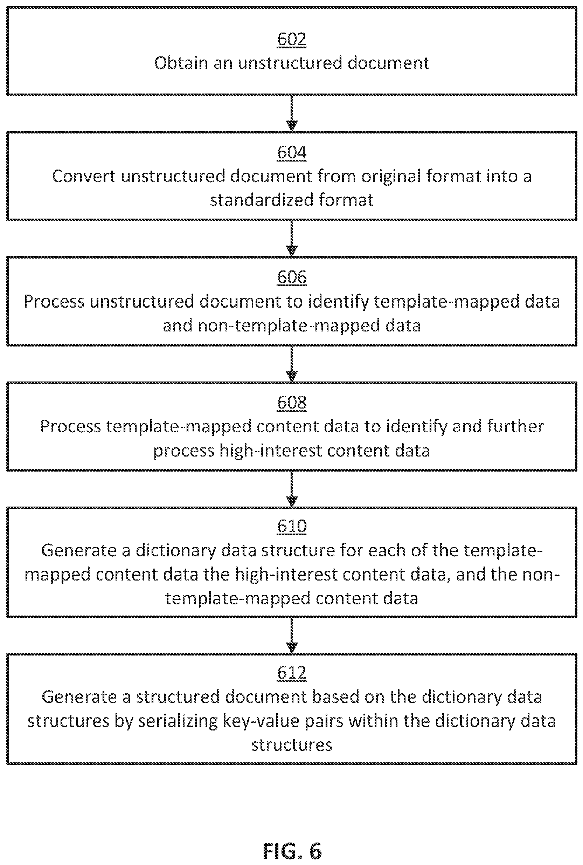

FIG. 6 depicts a flowchart illustrating a process 600 for abstraction of unstructured clinical trial health data according to various embodiments. The process 600 depicted in FIG. 6 is implemented by the architecture, systems, and techniques depicted in FIGS. 1-5.

At block 602, an unstructured document is obtained. The unstructured document 110 can be obtained by a data transformation system 105, such as those described in FIG. 1. The unstructured document 110 can be a protocol document, amendment history document, or other unstructured document that includes various types of content (e.g., template-mapped content 112, non-template-mapped content 116, and high-interest content 114) for a given event, such as a clinical trial. If the unstructured document 110 is a protocol document it can include any of the various types of content, but if the unstructured document 110 is an amendment history document it may only include non-template-mapped content 116.

At block 604, the unstructured document 110 is converted from an original format into a standardized format. The original format may be XML and the standardized format may be HTML. The data transformation system 105 can convert the unstructured document 110 by extracting a list of tags used to program the unstructured document 110 in the original format and mapping the list of tags to a corresponding list of tags defined by the standardized format. The content of the unstructured document 110 can then be converted from the original format into the standardized format based on the mapping.

At block 606, the unstructured document 110 is processed to identify template-mapped content data and non-template-mapped content data. The template-mapped content data can include a background section and inclusion and exclusion criteria, and the non-template-mapped content can include an amendment section. The template-mapped content data and the non-template mapped content data may be identified by the processes described in FIGS. 4 and 5.

At block 608, the template-mapped content data is processed to identify and further process high-interest content data. The high-interest content data can include an SOA and/or inclusion and exclusion criteria. The high-interest content data can be identified by the processes described in FIG. 4.

At block 610, a dictionary data structure 135a-c is generated for each of the template-mapped content data, the non-template-mapped content data, and the high-interest content data. The dictionary data structures 135a-c can be generated by writing the various types of content as key-value pairs. The keys and values for the key-value pairs can be based on the type of content, as described in FIGS. 4 and 5.

At block 612, a structured document 140 is generated based on the dictionary data structures 135a-c by serializing the key-value pairs within the dictionary data structures 135a-c. Each of the dictionary data structures 135a-c can be aggregated to generate the structured document 140. The key-value pairs can be serialized to data objects in an open standard file format (e.g., JSON). The structured document 140 may then be communicated to an end user, provided to a search engine, provided to an authoring tool, or provided to an analysis tool. A query may be performed for data using the structured document 140, a new document may be generated based on the various types of content in the structured document 140, and/or the structured document 140 may be analyzed to determine an insight.

### V. Additional Considerations

Some embodiments of the present disclosure include a system including one or more data processors. In some embodiments, the system includes a non-transitory computer readable storage medium containing instructions which, when executed on the one or more data processors, cause the one or more data processors to perform part or all of one or more methods and/or part or all of one or more processes disclosed herein. Some embodiments of the present disclosure include a computer-program product tangibly embodied in a non-transitory machine-readable storage medium, including instructions configured to cause one or more data processors to perform part or all of one or more methods and/or part or all of one or more processes disclosed herein.

The description provides preferred exemplary embodiments only, and is not intended to limit the scope, applicability or configuration of the disclosure. Rather, the description of the preferred exemplary embodiments will provide those skilled in the art with an enabling description for implementing various embodiments. It is understood that various changes may be made in the function and arrangement of elements without departing from the scope as set forth in the appended claims.

Specific details are given in the following description to provide a thorough understanding of the embodiments. However, it will be understood that the embodiments may be practiced without these specific details. For example, circuits, systems, networks, processes, and other components may be shown as components in block diagram form in order not to obscure the embodiments in unnecessary detail. In other instances, well-known circuits, processes, algorithms, structures, and techniques may be shown without unnecessary detail in order to avoid obscuring the embodiments.

## Claims

1. A computer-implemented method comprising:
obtaining, by a data transformation system (105), an unstructured document (110, 210) that includes various types of content for a given event, wherein the given event is a clinical trial;
converting, by the data transformation system (105), the unstructured document (110, 210) from an original format into a standardized format;
processing, by the data transformation system (105), the unstructured document (110, 210) in the standardized format to identify template-mapped content data (112), wherein the processing comprises:
determining whether each element in the unstructured document is a header (212), text, a table, or an image;
in response to determining the element is a header (212), determining a nesting level of the header and storing the header as a subheader (244) under a header or as an independent header (240) based on the nesting level; and
in response to determining the element is the text, the table, or the image, storing the text, the table, or the image under a latest processed header;
processing, by the data transformation system (105), the template-mapped content data (112) to identify high-interest content data (114);
generating, by the data transformation system (105), a dictionary data structure (135a-b) for each of the template-mapped content data (112) and the high-interest content data (114), wherein the dictionary data structure comprises key-value pairs, wherein the generating comprises:
writing the template-mapped content data (112) in the dictionary data structure as the key-value pairs, wherein the headers (212) are written as keys (242), and the subheaders, the text, the tables, and the images under the headers are written as values (244) associated with the keys (242); and
generating, by the data transformation system (105), a structured document (140) based on the dictionary data structure (135a-b) for each of the template-mapped content data (112) and the high-interest content data (114), wherein the generating the structured document comprises serializing the key-value pairs within the dictionary data structure to data objects in an open standard file format, and wherein the serializing comprises:
writing the key-value pairs to an object based data structure (235) comprising the data objects with properties, wherein the properties are the key-value pairs, each of the keys is a name of a property, and each of the values is a schema used to validate the property.

2. The computer-implemented method of claim 1, wherein the obtaining the unstructured document (110, 210), comprises:
obtaining a plurality of unstructured documents (201) for the given event;
collating protocol documents (504) and amendment history documents from the plurality of unstructured documents; and
retrieving the unstructured document (110) from the collated protocol documents (504).

3. The computer-implemented method of claim 2, wherein the collating the protocol documents (504) and the amendment history documents, comprises:
identifying the protocol documents (504) and the amendment history documents within the plurality of unstructured documents by matching a key word or phrase associated with the protocol documents or the amendment history documents to a word or phrase within a name or content of an unstructured document; and
grouping the identified protocol documents (540) and the amendment history documents into a subgroup of unstructured documents.

4. The computer-implemented method of claim 1, wherein the converting the unstructured document (110) into the standardized format, comprises:
extracting a list of tags from the unstructured document (110) based on a source markup language used to program the unstructured document, wherein the list of tags show a structure of content in the unstructured document, the content including headers (212), text, tables, images, or any combination thereof;
mapping the list of tags to a corresponding list of tags that are defined by a target markup language, and
converting the content of the unstructured document (110) from the original format into the standardized format based on the mapping between the list of tags and the corresponding list of tags defined by the target markup language.

5. The computer-implemented method of claim 4, further comprising:
extracting, by the data transformation system (105), the corresponding list of tags defined by the target markup language from the unstructured document (110) in the standardized format; and
removing, by the data transformation system (105), redundant tags and associated content.

6. The computer-implemented method of any one of claims 1, 4, or 5, wherein the processing to identify the high-interest content data (114) comprises:
parsing each table within the template-mapped content data (112) into a list of table elements;
searching the list of table elements to find an element associated with high-interest content (114);
in response to finding the element, searching the list of table elements to find another element associated with orientation of the table;
in response to finding the another element, determining the orientation of the table;
analyzing the table elements based on the orientation to identify procedures and cell content; and
storing the identified procedures and the cell content.

7. The computer-implemented method of claim 6, wherein:
the generating the dictionary data structure (135b) for the high-interest content data (114) comprises writing the high-interest content data in the dictionary data structure as the key-value pairs, wherein the procedures are written as keys, and the cell content are written as values associated with the keys; and
the serializing the key-value pairs comprises writing the key-value pairs to an object based data structure (235) comprising the data objects with properties, the properties are the key-value pairs, each of the keys is a name of a property, and each of the values is a schema used to validate the property.

8. The computer-implemented method of claim 7, wherein the analyzing the table elements is performed using a statistical based approach or a natural language processing model.

9. The computer-implemented method of any one of claims 1 or 2, further comprising:
processing, by the data transformation system (105), the unstructured document (110) to identify non-template-mapped content data (116); and
generating, by the data transformation system (105), a dictionary data structure (135c) for the non-template-mapped content data,
wherein the structured document (110) is generated based on the non-template-mapped content data (116), the template-mapped content data (112), and the high-interest content data (114).

10. The computer-implemented method of claim 9, wherein the processing the unstructured document (110) to identify the non-template-mapped content data (116) comprises:
parsing the unstructured document (110) to find a header-style paragraph containing a key word or phrase associated with non-template-mapped content (116);
in response to finding the header-style paragraph, collecting the non-template-mapped content (116) under the header-style paragraph until a style change in the non-template-mapped content is identified or another header-style paragraph is found; and
storing an identifier for the given event, the header-style paragraphs, and the non-template-mapped content (116) in a list as the non-template-mapped content data.

11. The computer-implemented method of claim 10, wherein:
the generating the dictionary data structure (135c) for the non-template-mapped content data (116) comprises writing the non-template-mapped content data in the dictionary data structure as the key-value pairs, wherein the identifier for the given event and the header-style paragraphs are written as keys, and the non-template-mapped content under the header-style paragraphs are written as values associated with the keys; and
the serializing the key-value pairs comprises writing the key-value pairs to an object based data structure (235) comprising the data objects with properties, the properties are the key-value pairs, each of the keys is a name of a property, and each of the values is a schema used to validate the property.

12. The computer-implemented method of claim 2, further comprising:
selecting, by the data transformation system (105), another unstructured document (110) from the amendment history documents;
processing, by the data transformation system (105), the another unstructured document (110) to identify non-template-mapped content data (116);
generating, by the data transformation system (105), the dictionary data structure (135c) for the non-template-mapped content data (116); and
generating, by the data transformation system (105), another structured document (140) based on the non-template-mapped content data (116), wherein the generating the another structured document comprises serializing the key-value pairs within the dictionary data structure (135c) to the data objects in the open standard file format.

13. The computer-implemented method of any one of claims 1-12, further comprising:
communicating, by the data transformation system (105), the structured document (140) to an end user; or
providing, by the data transformation system (105), the structured document (140) to a search engine, and performing, by the search engine, a query for data using the structured document; or
providing, by the data transformation system (105), the structured document (140) to an authoring tool, and generating, by the authoring tool, a new document based on the template-mapped content data (112), the high-interest content data (114), the non-template-mapped content data (116), or any combination thereof; or
providing, by the data transformation system (105), the structured document (140) to an analysis tool, and analyzing, by the analysis tool, the template-mapped content data (112), the high-interest content data (114), the non-template-mapped content data (116), or any combination thereof to determine an insight.

14. A system comprising:
one or more data processors; and
a non-transitory computer readable storage medium containing instructions which, when executed on the one or more data processors, cause the one or more data processors to perform the method of any preceding claim.

15. A computer-program product tangibly embodied in a non-transitory machine-readable storage medium, including instructions configured to cause one or more data processors to perform the method of any of claims 1 to 13.

## Patentansprüche

1. Computerimplementiertes Verfahren, umfassend:
Erhalten eines unstrukturierten Dokuments (110, 210), das verschiedene Arten von Inhalt für ein gegebenes Ereignis einschließt, mittels eines Datentransformationssystems (105), wobei das gegebene Ereignis eine klinische Studie ist;
Umwandeln des unstrukturierten Dokuments (110, 210) aus einem ursprünglichen Format in ein standardisiertes Format mittels des Datentransformationssystems (105);
Verarbeiten des unstrukturierten Dokuments (110, 210) in dem standardisierten Format mittels des Datentransformationssystems (105) zum Identifizieren von vorlagenzugeordneten Inhaltsdaten (112), wobei das Verarbeiten Folgendes umfasst:
Bestimmen, ob jedes Element in dem unstrukturierten Dokument ein Header (212), ein Text, eine Tabelle oder ein Bild ist;
als Reaktion auf das Bestimmen, dass das Element ein Header (212) ist, Bestimmen eines Verschachtelungsniveaus des Headers und Speichern des Headers als einen Subheader (244) unter einem Header oder als einen unabhängigen Header (240) basierend auf dem Verschachtelungsniveau; und
als Reaktion auf das Bestimmen, dass das Element der Text, die Tabelle oder das Bild ist, Speichern des Textes, der Tabelle oder des Bildes unter einem zuletzt bearbeiteten Header;
Verarbeiten der vorlagenzugeordneten Inhaltsdaten (112) mittels des Datentransformationssystems (105) zum Identifizieren von Inhaltsdaten von hohem Interesse (114);
Erzeugen einer Wörterbuchdatenstruktur (135a-b) für alle von den vorlagenzugeordneten Inhaltsdaten (112) und den Inhaltsdaten von hohem Interesse (114) mittels des Datentransformationssystems (105), wobei die Wörterbuchdatenstruktur Schlüsselwertpaare umfasst, wobei das Erzeugen Folgendes umfasst:
Schreiben der vorlagenzugeordneten Inhaltsdaten (112) in die Wörterbuchdatenstruktur als die Schlüsselwertpaare, wobei die Header (212) als Schlüssel (242) geschrieben werden und die Subheader, der Text, die Tabellen und die Bilder unter den Headern als Werte (244) in Verbindung mit den Schlüsseln (242) geschrieben werden; und
Erzeugen eines strukturierten Dokuments (140) mittels des Datentransformationssystems (105) basierend auf der Wörterbuchdatenstruktur (135a-b) für alle von den vorlagenzugeordneten Inhaltsdaten (112) und den Inhaltsdaten von hohem Interesse (114), wobei das Erzeugen des strukturierten Dokuments das Serialisieren der Schlüsselwertpaare innerhalb der Wörterbuchdatenstruktur auf Datenobjekte in einem Open-Standard-Dateiformat umfasst und wobei das Serialisieren Folgendes umfasst:
Schreiben der Schlüsselwertpaare auf eine objektbasierte Datenstruktur (235), die die die Datenobjekte mit Eigenschaften umfasst, wobei die Eigenschaften die Schlüsselwertpaare sind, jeder Schlüssel ein Name einer Eigenschaft ist und jeder der Werte ein Schema ist, das zum Validieren der Eigenschaft verwendet wird.

2. Computerimplementiertes Verfahren nach Anspruch 1, wobei das Erhalten des unstrukturierten Dokuments (110, 210) Folgendes umfasst:
Erhalten einer Vielzahl von unstrukturierten Dokumenten (201) für das gegebene Ereignis;
Vergleichen von Protokolldokumenten (504) und Änderungshistoriendokumenten aus der Vielzahl von unstrukturierten Dokumenten; und
Abrufen des unstrukturierten Dokuments (110) aus den verglichenen Protokolldokumenten (504).

3. Computerimplementiertes Verfahren nach Anspruch 2, wobei das Vergleichen der Protokolldokumente (504) und Änderungshistoriendokumente Folgendes umfasst:
Identifizieren der Protokolldokumente (504) und der Änderungshistoriendokumente innerhalb der Vielzahl von unstrukturierten Dokumenten durch Abgleichen eines Schlüsselwortes oder -ausdrucks in Verbindung mit den Protokolldokumenten oder den Änderungshistoriendokumenten mit einem Wort oder Ausdruck innerhalb eines Namens oder Inhalts eines unstrukturierten Dokuments; und
Gruppieren der identifizierten Protokolldokumente (540) und der Änderungshistoriendokumente in eine Untergruppe von unstrukturierten Dokumenten.

4. Computerimplementiertes Verfahren nach Anspruch 1, wobei das Umwandeln des unstrukturierten Dokuments (110) in das standardisierte Format Folgendes umfasst:
Extrahieren einer Liste von Tags aus dem unstrukturierten Dokument (110) basierend auf einer Quellenauszeichnungssprache, die zum Programmieren des unstrukturierten Dokuments verwendet wird, wobei die Liste von Tags eine Struktur von Inhalt in dem unstrukturierten Dokument zeigen, wobei der Inhalt Header (212), Text, Tabellen, Bilder oder eine Kombination davon einschließt;
Mapping der Liste von Tags mit einer entsprechenden Liste von Tags, die von einer Zielauszeichnungssprache definiert werden, und
Umwandeln des Inhalts des unstrukturierten Dokuments (110) aus dem ursprünglichen Format in das standardisierte Format basierend auf dem Mapping zwischen der Liste von Tags und der entsprechenden Liste von Tags, die von der Zielauszeichnungssprache definiert werden.

5. Computerimplementiertes Verfahren nach Anspruch 4, ferner umfassend:
Extrahieren der entsprechenden Liste von Tags, die von der Zielauszeichnungssprache definiert werden, aus dem unstrukturierten Dokument (110) in dem standardisierten Format mittels des Datentransformationssystems (105) und
Entfernen von überflüssigen Tags und damit verbundenem Inhalt mittels des Datentransformationssystems (105).

6. Computerimplementiertes Verfahren nach einem der Ansprüche 1, 4 oder 5, wobei das Verarbeiten zum Identifizieren der Inhaltsdaten von hohem Interesse (114) Folgendes umfasst:
Parsen jeder Tabelle innerhalb der vorlagenzugeordneten Inhaltsdaten (112) in eine Liste von Tabellenelementen;
Durchsuchen der Liste von Tabellenelementen, um ein Element zu finden, das mit Inhalt von hohem Interesse (114) in Verbindung steht;
als Reaktion auf das Finden des Elements, Durchsuchen der Liste von Tabellenelementen, um ein anderes Element zu finden, das mit Orientierung der Tabelle in Verbindung steht;
als Reaktion auf das Finden des anderen Elements, Bestimmen der Orientierung der Tabelle;
Analysieren der Tabellenelemente basierend auf der Orientierung, um Prozeduren und Zelleninhalt zu identifizieren; und
Speichern der identifizierten Prozeduren und des Zelleninhalts.

7. Computerimplementiertes Verfahren nach Anspruch 6, wobei:
das Erzeugen der Wörterbuchdatenstruktur (135b) für die Inhaltsdaten von hohem Interesse (114) das Schreiben der Inhaltsdaten von hohem Interesse in die Wörterbuchdatenstruktur als die Schlüsselwertpaare umfasst, wobei die Prozeduren als Schlüssel geschrieben werden und der Zelleninhalt als Werte in Verbindung mit den Schlüsseln geschrieben wird; und
das Serialisieren der Schlüsselwertpaare das Schreiben der Schlüsselwertpaare auf eine objektbasierte Datenstruktur (235) umfasst, die die Datenobjekte mit Eigenschaften umfasst, wobei die Eigenschaften die Schlüsselwertpaare sind, wobei jeder der Schlüssel ein Name einer Eigenschaft ist und jeder der Werte ein Schema ist, das zum Validieren der Eigenschaft verwendet wird.

8. Computerimplementiertes Verfahren nach Anspruch 7, wobei das Analysieren der Tabellenelemente unter Anwendung eines statistisch basierten Ansatzes oder eines Verarbeitungsmodells mit natürlicher Sprache durchgeführt wird.

9. Computerimplementiertes Verfahren nach einem der Ansprüche 1 oder 2, ferner umfassend:
Verarbeiten des unstrukturierten Dokuments (110) mittels des Datentransformationssystems (105), um nicht vorlagenzugeordnete Inhaltsdaten (116) zu identifizieren; und
Erzeugen einer Wörterbuchdatenstruktur (135c) für die nicht vorlagenzugeordneten Inhaltsdaten mittels des Datentransformationssystems (105),
wobei das strukturierte Dokument (110) basierend auf den nicht vorlagenzugeordneten Inhaltsdaten (116), den vorlagenzugeordneten Inhaltsdaten (112) und den Inhaltsdaten von hohem Interesse (114) erzeugt wird.

10. Computerimplementiertes Verfahren nach Anspruch 9, wobei das Verarbeiten des unstrukturierten Dokuments (110) zum Identifizieren der nicht vorlagenzugeordneten Inhaltsdaten (116) Folgendes umfasst:
Parsen des unstrukturierten Dokuments (110), um einen Header-Stil-Absatz zu finden, der ein(en) Schlüsselwort oder -ausdruck in Verbindung mit nicht vorlagenzugeordnetem Inhalt (116) enthält;
als Reaktion auf das Finden des Header-Stil-Absatzes, Sammeln des nicht vorlagenzugeordneten Inhalts (116) unter dem Header-Stil-Absatz, bis eine Stiländerung des nicht vorlagenzugeordneten Inhalts identifiziert oder ein anderer Header-Stil-Absatz gefunden wird; und
Speichern einer Kennzeichnung für das gegebene Ereignis, die Header-Stil-Absätze und den nicht vorlagenzugeordneten Inhalt (116) in einer Liste als die nicht vorlagenzugeordneten Inhaltsdaten.

11. Computerimplementiertes Verfahren nach Anspruch 10, wobei:
das Erzeugen der Wörterbuchdatenstruktur (135c) für die nicht vorlagenzugeordneten Inhaltsdaten (116) das Schreiben der nicht vorlagenzugeordneten Inhaltsdaten in die Wörterbuchdatenstruktur als die Schlüsselwertpaare umfasst, wobei die Kennzeichnung für das gegebene Ereignis und die Header-Stil-Absätze als Schlüssel geschrieben wird und der nicht vorlagenzugeordnete Inhalt unter den Header-Stil-Absätzen als Werte in Verbindung mit den Schlüsseln geschrieben wird; und
das Serialisieren der Schlüsselwertpaare das Schreiben der Schlüsselwertpaare auf eine objektbasierte Datenstruktur (235) umfasst, die die Datenobjekte mit Eigenschaften umfasst, wobei die Eigenschaften die Schlüsselwertpaare sind, wobei jeder der Schlüssel ein Name einer Eigenschaft ist und jeder der Werte ein Schema ist, das zum Validieren der Eigenschaft verwendet wird.

12. Computerimplementiertes Verfahren nach Anspruch 2, ferner umfassend:
Auswählen eines anderen unstrukturierten Dokuments (110) aus den Änderungshistoriendokumenten mittels des Datentransformationssystems (105);
Verarbeiten des anderen unstrukturierten Dokuments (110) mittels des Datentransformationssystems (105), um nicht vorlagenzugeordnete Inhaltsdaten (116) zu identifizieren;
Erzeugen der Wörterbuchdatenstruktur (135c) für die nicht vorlagenzugeordneten Inhaltsdaten (116) mittels des Datentransformationssystems (105); und
Erzeugen eines anderen strukturierten Dokuments (140) basierend auf den nicht vorlagenzugeordneten Inhaltsdaten (116) mittels des Datentransformationssystems (105), wobei das Erzeugen des anderen strukturierten Dokuments das Serialisieren der Schlüsselwertpaare innerhalb der Wörterbuchdatenstruktur (135c) auf die Datenobjekte in dem Open-Standard-Dateiformat umfasst.

13. Computerimplementiertes Verfahren nach einem der Ansprüche 1-12, ferner umfassend:
Übermitteln des strukturierten Dokuments (140) mittels des Datentransformationssystems (105) an einen Endbenutzer; oder
Bereitstellen des strukturierten Dokuments (140) mittels des Datentransformationssystems (105) einer Suchmaschine und Ausführen einer Abfrage für Daten unter Verwendung des strukturierten Dokuments mittels der Suchmaschine; oder
Bereitstellen des strukturierten Dokuments (140) mittels des Datentransformationssystems (105) einem Autorisierungs-Tool und Erzeugen eines neuen Dokuments basierend auf den vorlagenzugeordneten Inhaltsdaten (112), den Inhaltsdaten von hohem Interesse (114), den nicht vorlagenzugeordneten Inhaltsdaten (116) oder einer Kombination davon mittels des Autorisierungs-Tools; oder
Bereitstellen des strukturierten Dokuments (140) mittels des Datentransformationssystems (105) einem Analyse-Tool und Analysieren der vorlagenzugeordneten Inhaltsdaten (112), der Inhaltsdaten von hohem Interesse (114), der nicht vorlagenzugeordneten Inhaltsdaten (116) oder einer Kombination davon mittels des Analyse-Tools, um einen Einblick zu bestimmen.

14. System, umfassend:
einen oder mehrere Datenprozessor(en); und
ein nicht flüchtiges, computerlesbares Speichermedium, das Anweisungen enthält, die beim Ausführen auf dem einen oder den mehreren Datenprozessor(en) den einen oder die mehreren Datenprozessor(en) veranlassen, das Verfahren nach einem vorstehenden Anspruch auszuführen.

15. Computerprogrammprodukt, das in einem nicht flüchtigen, maschinenlesbaren Speichermedium körperlich ausgeführt ist und Anweisungen einschließt, die dafür ausgebildet sind, einen oder mehrere Datenprozessor(en) zu veranlassen, das Verfahren nach einem der Ansprüche 1 bis 13 auszuführen.

## Revendications

1. Procédé mis en œuvre par ordinateur comprenant :
l'obtention, par un système de transformation de données (105), d'un document non structuré (110, 210) qui inclut divers types de contenu pour un événement donné, dans lequel l'événement donné est un essai clinique ;
la conversion, par le système de transformation de données (105), du document non structuré (110, 210) d'un format d'origine en un format normalisé ;
le traitement, par le système de transformation de données (105), du document non
structuré (110, 210) en format normalisé pour identifier des données de contenu mappé à un modèle (112), dans lequel le traitement comprend :
la détermination si chaque élément dans le document non structuré est un en-tête (212), un texte, un tableau ou une image ;
en réponse à la détermination que l'élément est un en-tête (212), la détermination d'un niveau d'imbrication de l'en-tête et le stockage de l'en-tête en tant que sous-en-tête (244) sous un en-tête ou en tant qu'en-tête indépendant (240) en se basant sur le niveau d'imbrication ; et
en réponse à la détermination que l'élément est le texte, le tableau ou l'image, le stockage du texte, du tableau ou de l'image sous un en-tête traité le plus récent ;
le traitement, par le système de transformation de données (105), des données de contenu mappé à un modèle (112) pour identifier des données de contenu d'intérêt élevé (114) ;
la génération, par le système de transformation de données (105), d'une structure de données de dictionnaire (135a-b) pour chacune des données de contenu mappé à un modèle (112) et des données de contenu d'intérêt élevé (114), dans lequel la structure de données de dictionnaire comprend des paires clé-valeur, dans lequel la génération comprend :
l'écriture des données de contenu mappé à un modèle (112) dans la structure de données de dictionnaire en tant que paires clé-valeur, dans lequel les en-têtes (212) sont écrits en tant que clés (242), et les sous-en-têtes, le texte, les tableaux et les images sous les en-têtes sont écrits en tant que valeurs (244) associées aux clés (242) ; et
la génération, par le système de transformation de données (105), d'un document structuré (140) en se basant sur la structure de données de dictionnaire (135a-b) pour chacune des données de contenu mappé à un modèle (112) et des données de contenu d'intérêt élevé (114), dans lequel la génération du document structuré comprend la sérialisation des paires clé-valeur au sein de la structure de données de dictionnaire en objets de données dans un format de fichier standard ouvert, et dans lequel la sérialisation comprend :
l'écriture des paires clé-valeur sur une structure de données basée sur objets (235) comprenant les objets de données avec des propriétés, dans lequel les propriétés sont les paires clé-valeur, chacune des clés est le nom d'une propriété, et chacune des valeurs est un schéma utilisé pour valider la propriété.

2. Procédé mis en œuvre par ordinateur selon la revendication 1, dans lequel l'obtention du document non structuré (110, 210) comprend :
l'obtention d'une pluralité de documents non structurés (201) pour l'événement donné ;
le rassemblement de documents de protocole (504) et de documents d'historique de modification à partir de la pluralité de documents non structurés ; et
la récupération du document non structuré (110) à partir des documents de protocole rassemblés (504).

3. Procédé mis en œuvre par ordinateur selon la revendication 2, dans lequel le rassemblement des documents de protocole (504) et des documents d'historique de modification comprend :
l'identification des documents de protocole (504) et des documents d'historique de modification au sein de la pluralité de documents non structurés en faisant correspondre un mot-clé ou une phrase-clé associée aux documents de protocole ou aux documents d'historique de modification à un mot ou une phrase au sein d'un nom ou d'un contenu d'un document non structuré ; et
le regroupement des documents de protocole (540) identifiés et des documents d'historique de modification en un sous-groupe de documents non structurés.

4. Procédé mis en œuvre par ordinateur selon la revendication 1, dans lequel la conversion du document non structuré (110) en format normalisé comprend :
l'extraction d'une liste de balises à partir du document non structuré (110) en se basant sur un langage de balisage source utilisé pour programmer le document non structuré, dans lequel la liste de balises affiche une structure de contenu dans le document non structuré, le contenu incluant des en-têtes (212), du texte, des tableaux, des images, ou une quelconque combinaison de ceux-ci ;
le mappage de la liste de balises à une liste correspondante de balises qui sont définies par un langage de balisage cible, et
la conversion du contenu du document non structuré (110) du format d'origine en format normalisé en se basant sur le mappage entre la liste de balises et la liste correspondante de balises définies par le langage de balisage cible.

5. Procédé mis en œuvre par ordinateur selon la revendication 4, comprenant en outre :
l'extraction, par le système de transformation de données (105), de la liste correspondante de balises définies par le langage de balisage cible du document non structuré (110) en format normalisé ; et
le retrait, par le système de transformation de données (105), des balises redondantes et du contenu associé.

6. Procédé mis en œuvre par ordinateur selon l'une quelconque des revendications 1, 4 ou 5, dans lequel le traitement pour identifier les données de contenu d'intérêt élevé (114) comprend :
l'analyse syntaxique de chaque tableau au sein des données de contenu mappé à un modèle (112) en une liste d'éléments de tableau ; la recherche dans la liste d'éléments de tableau pour trouver un élément associé au contenu d'intérêt élevé (114) ;
en réponse au fait de trouver l'élément, la recherche dans la liste d'éléments de tableau pour trouver un autre élément associé à l'orientation du tableau ;
en réponse au fait de trouver l'autre élément, la détermination de l'orientation du tableau ;
l'analyse des éléments de tableau en se basant sur l'orientation pour identifier les procédures et le contenu des cellules ; et
le stockage des procédures et du contenu des cellules identifiés.

7. Procédé mis en œuvre par ordinateur selon la revendication 6, dans lequel :
la génération de la structure de données de dictionnaire (135b) pour les données de contenu d'intérêt élevé (114) comprend l'écriture des données de contenu d'intérêt élevé dans la structure de données de dictionnaire en tant que paires clé-valeur, dans lequel les procédures sont écrites en tant que clés, et le contenu des cellules est écrit en tant que valeurs associées aux clés ; et
la sérialisation des paires clé-valeur comprend l'écriture des paires clé-valeur sur une structure de données basée sur objets (235) comprenant les objets de données avec des propriétés, les propriétés sont les paires clé-valeur, chacune des clés est le nom d'une propriété, et chacune des valeurs est un schéma utilisé pour valider la propriété.

8. Procédé mis en œuvre par ordinateur selon la revendication 7, dans lequel l'analyse des éléments de tableau est réalisée en utilisant une approche de type statistique ou un modèle de traitement de langage naturel.

9. Procédé mis en œuvre par ordinateur selon l'une quelconque des revendications 1 ou 2, comprenant en outre :
le traitement, par le système de transformation de données (105), du document non structuré (110) pour identifier les données de contenu non mappé à un modèle (116) ; et
la génération, par le système de transformation de données (105), d'une structure de données de dictionnaire (135c) pour les données de contenu non mappé à un modèle,
dans lequel le document structuré (110) est généré en se basant sur les données de contenu non mappé à un modèle (116), les données de contenu mappé à un modèle (112) et les données de contenu d'intérêt élevé (114).

10. Procédé mis en œuvre par ordinateur selon la revendication 9, dans lequel le traitement du document non structuré (110) pour identifier les données de contenu non mappé à un modèle (116) comprend :
l'analyse syntaxique du document non structuré (110) pour trouver un paragraphe de style en-tête contenant un mot-clé ou une phrase-clé associée à un contenu non mappé à un modèle (116) ;
en réponse au fait de trouver le paragraphe de style en-tête, le recueil du contenu non mappé à un modèle (116) sous le paragraphe de style en-tête jusqu'à ce qu'un changement de style dans le contenu non mappé à un modèle soit identifié ou qu'un autre paragraphe de style en-tête soit trouvé ; et
le stockage d'un identifiant pour l'événement donné, les paragraphes de style en-tête et le contenu non mappé à un modèle (116) dans une liste en tant que données de contenu non mappé à un modèle.

11. Procédé mis en œuvre par ordinateur selon la revendication 10, dans lequel :
la génération de la structure de données de dictionnaire (135c) pour les données de contenu non mappé à un modèle (116) comprend l'écriture des données de contenu non mappé à un modèle dans la structure de données de dictionnaire en tant que paires clé-valeur, dans lequel l'identifiant pour l'événement donné et les paragraphes de style en-tête sont écrits en tant que clés, et le contenu non mappé à un modèle sous les paragraphes de style en-tête est écrit en tant que valeurs associées aux clés ; et
la sérialisation des paires clé-valeur comprend l'écriture des paires clé-valeur sur une structure de données basée sur objets (235) comprenant les objets de données avec des propriétés, les propriétés sont les paires clé-valeur, chacune des clés est le nom d'une propriété, et chacune des valeurs est un schéma utilisé pour valider la propriété.

12. Procédé mis en œuvre par ordinateur selon la revendication 2, comprenant en outre : la sélection, par le système de transformation de données (105), d'un autre document non structuré (110) parmi les documents d'historique de modification ;
le traitement, par le système de transformation de données (105), de l'autre document non structuré (110) pour identifier les données de contenu non mappé à un modèle (116) ;
la génération, par le système de transformation de données (105), de la structure de données de dictionnaire (135c) pour les données de contenu non mappé à un modèle (116) ; et
la génération, par le système de transformation de données (105), d'un autre document structuré (140) en se basant sur les données de contenu non mappé à un modèle (116), dans lequel la génération de l'autre document structuré comprend la sérialisation des paires clé-valeur au sein de la structure de données de dictionnaire (135c) en objets de données dans le format de fichier standard ouvert.

13. Procédé mis en œuvre par ordinateur selon l'une quelconque des revendications 1 à 12, comprenant en outre :
la communication, par le système de transformation de données (105), du document structuré (140) à un utilisateur final ; ou
la fourniture, par le système de transformation de données (105), du document structuré (140) à un moteur de recherche, et la réalisation, par le moteur de recherche, d'une requête de données en utilisant le document structuré ; ou
la fourniture, par le système de transformation de données (105), du document structuré (140) à un outil de création, et la génération, par l'outil de création, d'un nouveau document basé sur les données de contenu mappé à un modèle (112), les données de contenus d'intérêt élevé (114), les données de contenu non mappé à un modèle (116), ou une quelconque combinaison de celles-ci ; ou
la fourniture, par le système de transformation de données (105), du document structuré (140) à un outil d'analyse, et l'analyse, par l'outil d'analyse, des données de contenu mappé à un modèle (112), des données de contenu d'intérêt élevé (114), des données de contenu non mappé à un modèle (116), ou d'une quelconque combinaison de celles-ci pour déterminer un aperçu.

14. Système comprenant :
un ou plusieurs processeurs de données ; et
un support de stockage lisible par ordinateur non transitoire contenant des instructions qui, lorsqu'elles sont exécutées sur le ou les processeurs de données, amènent le ou les processeurs de données à réaliser le procédé selon une quelconque revendication précédente.

15. Produit de programme informatique intégré de façon tangible dans un support de stockage lisible par machine non transitoire, comprenant des instructions configurées pour amener un ou plusieurs processeurs de données à réaliser le procédé selon l'une quelconque des revendications 1 à 13.
